# FASCICULE DE BREVET EUROPEEN

(11) **EP 2 706 980 B1**
(45) Date de publication et mention de la délivrance du brevet: **16.11.2016**
(21) Numéro de dépôt: 12725128.8
(22) Date de dépôt: 09.05.2012
(51) Int. Cl.: A61K 8/92, A61K 8/97, A61Q 19/08

(54) **COMPOSITION COSMÉTIQUE OU DERMATOLOGIQUE À BASE D'EXTRAITS D'IMMORTELLE ET SON UTILISATION**
KOSMETISCHE ODER DERMATOLOGISCHE ZUSAMMENSETZUNG AUS IMMORTELLE-EXTRAKTEN UND IHRE VERWENDUNG
COSMETIC OR DERMATOLOGICAL COMPOSITION MADE FROM EXTRACTS OF EVERLASTING AND USE THEREOF

(30) Priorité: 09.05.2011 FR 1153960
(43) Date de publication de la demande: 19.03.2014
(73) Titulaire: Laboratoires M & L, 04100 Manosque (FR)
(72) Inventeur: DEVILARD, Elisabeth, F-04130 Volx (FR); TOUREL, Cécile, F-13100 Aix En Provence (FR); PIERRISNARD, Laure, F-04130 Volx (FR); PIERRISNARD, Jean-Louis, F-04700 Oraison (FR)
(74) Mandataire: Novagraaf Technologies
(86) Numéro de dépôt international: PCT/FR2012/051028
(87) Numéro de publication internationale: WO 2012/153064

(56) Documents cités:
- FR-A1- 2 819 718
- FR-A1- 2 829 145
- FR-A1- 2 868 307
- FR-A1- 2 903 014
- DATABASE GNDP [Online] Mintel; avril 2011 (2011-04), "Brightening Moisture Mask", XP002666699, Database accession no. 1532807
- DATABASE GNDP [Online] Mintel; mars 2011 (2011-03), "Sublime Re-Densifying Night Cream", XP002666700, Database accession no. 1529942

## Description

### Domaine technique

La présente invention se rapporte à une composition cosmétique ou dermatologique anti-âge.

La présente invention se rapporte également à une méthode de traitement cosmétique anti-âge.

La présente invention trouve notamment une application dans le domaine cosmétique.

Dans la description ci-dessous, les références entre crochets ([]) renvoient à la liste des références présentées à la fin du texte.

### Etat de la technique

La peau est un organe d'échange. Son aspect visuel mais aussi tactile occupe une place très importante, particulièrement dans notre société occidentale. La peau est le révélateur du vieillissement humain.

Le vieillissement cutané peut entraîner une altération de perception de l'image corporelle avec, à terme, des symptômes dysmorphophobiques ou dépressifs tel que décrit dans Koblenzer CS. Psychologie aspects of aging and the skin. Clinics Dermatol 1996;14:171-7. [1]. Par ailleurs, les personnes ayant des signes de vieillissement cutané sont souvent perçues par notre société comme étant en mauvaise santé et incapables d'initiatives (Koblenzer CS. Psychologic aspects of aging and the skin. Clinics Dermatol 1996;14:171-7. [1]). Ainsi, la demande thérapeutique afin de prévenir le vieillissement cutané est aujourd'hui de plus en plus forte. Pour y répondre, une meilleure connaissance de sa physiopathologie, mais aussi de ses conséquences cliniques, est nécessaire. Comme tous les autres organes, la peau est soumise au vieillissement. Les facteurs chronologiques, génétiques et les facteurs endocriniens (Pierard GE. The quandary of climacteric skin ageing. Dermatology 1996;193:273-4.[3]), par exemple les bouleversements post-ménopausiques chez la femme, déterminent le vieillissement cutané intrinsèque.

La peau est également soumise à des agressions extrinsèques qui, au fil du temps, amplifient le phénomène de vieillissement intrinsèque. Le rôle des rayons ultraviolets (UV) est majeur. Ils sont responsables du photo-vieillissement. Les rayons UVA sont certainement au moins aussi nocifs que les rayons UVB tel qu'indiqué dans Petersen MJ, Hansen C, Craig S. Ultraviolet A irradiation stimulates collagenase production in cultured human fibroblasts. J Invest Dermatol 1992;99:440-4. [2].

Les facteurs environnementaux, au premier rang desquels le tabac et l'alcool, contribuent aussi au vieillissement cutané. Les autres rayonnements (infrarouges, rayons-X, rayons gamma, mais aussi probablement la lumière visible), la gravité interviennent dans une moindre mesure.

### A/ Le vieillissement intrinsèque :

Les mécanismes biologiques sont liés à un déséquilibre entre phénomènes de dégradation cellulaire (formes actives de l'oxygène, produits terminaux de la glycation etc.) et systèmes de réparation (anti-oxydants, enzymes de réparation de l'ADN, enzymes protéosomales, protéases, protéinases, phospholipases et acyltransférases, enzymes impliquées dans la stabilité des liaisons etc.). Les mécanismes de réparation de l'ADN jouent un rôle majeur dans la lutte contre le vieillissement. Plusieurs systèmes enzymatiques coopèrent pour réparer les mutations spontanément générées par l'organisme ou induites par des agressions extérieures (ex : rayonnement UV).

### B/ Le vieillissement extrinsèque :

Le rayonnement UV est le principal facteur responsable du vieillissement extrinsèque. Son mécanisme d'action est double : soit par interaction directe sur l'ADN cellulaire (interférence due en priorité aux UVB) soit de façon indirecte par le biais des formes actives de l'oxygène qui est le mode d'action principal des UVA (West MD. The cellular and molecular biology of skin aging. Arch dermatol 1994;130:87-95. [4]; Meydani M. Nutrition interventions in aging and age-associated disease. Ann N Y Acad Sci 2001;928:226-35 [5] ; Stege H, Roza L, Vink A et al. Enzyme plus light therapy to repair immunosuppressive effects on human skin damaged by ultraviolet B-radiation. Proc Natl Acad Sci USA 2000; 97:179-85. [6] ; Jurkiewicz BA, Buettner GR. Ultraviolet light-induced free radical formation in the skin: An electron paramagnetic resonance study. Photochem Photobiol 1994; 59:1-4. [7]; Grether-Beck S, Oliazola-Horn S, Schmitt H et al. Activation of transcriptor factor AP-2 mediates UVA radiation- and singlet oxygen-induced expression of the human intercellular adhésion molecule-1 gene. Proc Natl Acad Sci USA 1996; 93:14586-91. [8]).

En interagissant directement avec l'ADN, les UV induisent la formation de photo-produits (notamment des dimères de thymidine pour les UVB) pouvant conduire à des mutations, à l'initiation d'une carcinogenèse, voire à la mort cellulaire. Lorsque les doses d'UV reçues sont trop importantes, l'activation de la protéine p53 permet d'entraîner la mort du kératinocyte par phénomène d'apoptose.

Les radiations UV augmentent aussi l'expression de métalloprotéinases (MP) dans l'épiderme et le derme (ces enzymes dégradent le collagène et les autres macromolécules de la matrice extracellulaire dermique).

Une exposition solaire de 5 à 15 minutes seulement, à midi, entraîne la production de collagénase, de stromélysine-1 et de gélatinase de 92kDa. La collagénase hydrolyse les fibres collagènes, puis la stromélysine- 1 et la gélatinase de 92kd finissent ensuite de les dégrader. Le rôle spécifique du rayonnement UVA est aujourd'hui mieux connu. Il induit notamment l'expression de gènes dans les kératinocytes, responsables des phénomènes de photo-vieillissement et de photocarcinogénèse. Les rayons UVA agissent par le biais des formes actives de l'oxygène qui induisent entre autres la formation de céramides. Ces céramides entraînent à leur tour l'expression des protéines d'adhésion ICAM-1 et activent le facteur de transcription AP-2 qui joue probablement un rôle central dans le phénomène de photo-vieillissement (Fisher GJ, Wang ZQ, Datta SC, Varani J, Kang S, Voorhees JJ. Pathophysiology of premature skin aging induced by ultraviolet light. N Engl J Med 1997;337:1419-28. [9]). Un autre mode d'action des UVA impliquerait des mutations de l'ADN mitochondrial (ADNmt) également induites par les radicaux libres. Les mécanismes d'action des autres facteurs extrinsèques sont encore mal connus. Le tabac aggraverait les effets des rayonnements UV par l'induction de MP de type 1 qui diminuerait la teneur en collagène de la matrice extracellulaire dermique (Lanmann C, Bergmann J, Harisson G, Young AR. Matrix metalloproteinase-1 and skin aging in smokers. Lancet 2001;357:935-7. [10]). Une autre hypothèse suggère que la perte en fibres élastiques et non pas en collagène serait responsable des rides faciales chez les fumeurs (Fenske NA, Lober CW. Structural and functional changes of normal aging skin. J Am Acad Dermatol 1986;15:571-85.

### C/Conséquences histologiques, cliniques et fonctionnelles

Le vieillissement intrinsèque et le vieillissement extrinsèque additionnent donc leurs effets délétères sur toutes les structures cutanées. La part respective des modifications entraînées par le vieillissement intrinsèque ou extrinsèque est souvent difficilement individualisable de part l'existence de données bibliographiques parfois contradictoires. Avec l'âge, les différentes structures cutanées sont modifiées à la fois sur le plan morphologique et fonctionnel. Les principales conséquences sont un déclin des fonctions de défense, de cicatrisation, de perception et de thermo-régulation ainsi que l'installation d'un état inflammatoire chronique correspondant à une augmentation du nombre de macrophages et d'une sécheresse cutanée persistante (Harvell JD, Maibach HI. Percutaneous absorption and inflammation in aged skin: a review. J Am Acad Dermatol 1994;31:1015-21.

Sur le plan morphohistologique, le vieillissement cutané intrinsèque se manifeste par une atrophie du derme, c'est-à-dire une réduction du nombre de fibroblastes et cellules mastocytaires, et de l'épiderme. Au niveau de l'épiderme, le renouvellement (turn-over) de l'épiderme passe de 21 jours à 40 jours d'où l'impact négatif sur la cicatrisation et la réparation tissulaire (Grove GL, Kligman AM. Age-associated changes in human epidermal cell renewal. J Gerontol 38:137-42. [13]). Les kératinocytes voient leur taille diminuer et leur nombre se réduire. Le renouvellement cellulaire se ralentit et la cohésion entre le derme et l'épiderme s'atténue. Au niveau du derme, les fibres de collagène se brisent, leur renouvellement naturel devient plus difficile, l'élastine perd de son élasticité. Le derme perd peu à peu de sa densité, il s'amincie, les rides et ridules apparaissent.

Le vieillissement extrinsèque entraine quand à lui une réduction à la fois du nombre des fibres de collagène et impacte leur qualité. Il provoque l'accumulation de fibres élastiques de mauvaise qualité, réduisant ainsi l'élasticité de la peau. Comme le vieillissement intrinsèque, il ralentit la prolifération des cellules. Enfin, il réduit l'uniformité de la pigmentation, ce qui donne à la peau un aspect plus terne et âgé.

De nombreuses compositions anti-âges sont connues dans l'état de la technique. Le brevet FR 2 903 014 décrit une composition cosmétique comprenant en outre une huile essentielle d'hélichryse (i.e. immortelle) obtenue par distillation à la vapeur d'eau des sommités fleuries. Cette composition permet de stimuler la production d'énergie et la prolifération des kératinocytes, d'avoir une activité anti-radicalaire, de stimuler la micro-circulation et donc d'effectuer un traitement en profondeur. Après application sur la peau d'une composition à base d'immortelle selon l'exemple 9 (crème très précieuse), celle-ci apparaît plus lisse, plus ferme, régénérée et les rides sont atténuées.

Le brevet FR 2 829 145 décrit une composition cosmétique comprenant une huile essentielle d'immortelle extraite des sommités fleuries d'Helichrysum italicum, ainsi que l'utilisation cosmétique de cette huile essentielle. La dite huile a une activité anti-radicalaire élevée, permet de stimuler la micro-circulation, d'accroître l'échange cellulaire, de revitaliser et de dynamiser la biologie cutanée. Par conséquent, les propriétés anti-rides remarquable, une bonne fermeté et un lissage cutané sont observés.

Toutefois, ces compositions ne permettent pas de traiter efficacement à la fois le vieillissement intrinsèque et extrinsèque, ni ne permettent de traiter les causes de ce vieillissement.

Il existe donc un réel besoin de trouver un nouveau composé, composition et/ou un moyen de traitement efficace du vieillissement cutané et/ou des causes à l'origine de ce vieillissement palliant l'ensemble des défauts, inconvénients et obstacles de l'art antérieur cités ci-dessus.

### Exposé de l'invention

La présente invention a précisément pour but de répondre à ce besoin en fournissant une composition cosmétique ou dermatologique comprenant :
- au moins un extrait aqueux d'immortelle,
- au moins un extrait huileux de sommités fleuries d'immortelle, et
- au moins de l'huile essentielle d'immortelle.

La présente invention se rapporte également à l'utilisation cosmétique et/ou dermatologique de cette composition.

Les inventeurs à l'origine de la présente sont en effet les tout premiers à avoir mis en évidence, de manière tout à fait inattendue, que cette composition constituée de trois extraits d'immortelle permet en outre, avantageusement et de manière cumulée de :
- stimuler la prolifération et la différenciation des kératinocytes,
- stimuler la desquamation et la consolidation de la fonction barrière et de la jonction dermo-épidermique (JDE).

En outre, Les travaux réalisés par les inventeurs dans le cadre de l'invention ont permis de caractériser chacun de ces extraits et de mettre en évidence leur complémentarité et synergie d'action.

Dans la présente par « immortelle » on entend une espèce de plantes à fleurs de la famille des Asteraceae et du genre Helichrysum. En particulier, il peut s'agir de *l'Hélicrysum italicum* qui peut être présente tout autour de la Méditerranée. Il peut s'agir par exemple de l'immortelle décrite dans la demande de brevet français n°01/11224.

Dans la présente par « extrait aqueux » on entend tout extrait aqueux obtenu par tout procédé connu de l'homme du métier. Par exemple, il peut être obtenu par extraction aqueuse, par hydrodistillation, extraction hydroglycérinée ou hydro-alcoolique.

Selon l'invention, l'extrait aqueux d'immortelle peut être tout extrait aqueux d'immortelle connu de l'homme du métier. Il peut s'agir par exemple d'un extrait aqueux biologique Immortelle, par exemple un extrait d'immortelle biologique de Corse de la société Greentech.

Dans la présente, l'extrait aqueux peut être obtenu par exemple à partir d'une plante et/ou d'une partie d'une plante. Il peut s'agir par exemple d'un extrait aqueux obtenu à partir de fleurs ou sommités fleuries, feuilles, graines, racines, tiges. Il peut s'agir par exemple d'un extrait obtenu à partir de fleurs entières.

Selon l'invention l'extrait aqueux peut comprendre par exemple de 0,1 à 0,3 % de sucres, par exemple 0,2% en poids de sucres, de 1 à 2 ppm de vitamine E, par exemple 1,7 ppm de vitamine E et de 0,2 à 0,5% en poids de polyphénols, par exemple 0,44% en poids de polyphénols dont 50ppm d'acide caféique.

Avantageusement, selon l'invention, l'extrait aqueux d'immortelle permet notamment de stimuler la loricrine, favorisant la restructuration de l'épiderme et un meilleur lissage de surface.

Dans la présente par « extrait huileux » on entend un extrait riche en acide gras et/ou en matière grasse et/ou un extrait non hydrosoluble.

Selon l'invention, l'extrait huileux peut être tout extrait huileux obtenu par tout procédé connu de l'homme du métier. Par exemple, il peut être obtenu par extraction huileuse par micro-ondes, par hyperfréquence, par extraction supercritique, par macération dans un solvant huileux, par extraction huileuse assistée par ultra sons. Il peut s'agir par exemple d'un extrait huileux biologique d'immortelle par extraction micro-ondes assistée par ultra sons selon un procédé comprenant une première étape de broyage de la sommité fleurie d'immortelle suivie d'une extraction assistée par ultrasons et par micro-ondes, puis une clarification et enfin une étape de filtration afin de récupérer l'extrait huileux.

Dans la présente, l'extrait huileux peut être obtenu à partir d'une plante et/ou d'une partie d'une plante. Il peut s'agir par exemple d'un extrait huileux obtenus à partir de fleurs ou sommités fleuries, feuilles, graines, racines, tiges, péricarpes.

Dans la présente, l'extrait huileux d'immortelle peut comprendre de 0,1 à 0,3% en poids de sucres, par exemple 0,2% de sucres, de 0,04 à 0,06% en poids de vitamine E, par exemple 0,051% de vitamine E, de 99% à 99,9% en poids d'acides gras, par exemple 99,8% en poids d'acides gras, dont de 80 à 85% d'acide oléique, par exemple 83,4% d'acide oléique, de 6 à 8% d'acide linoléique (oméga-6), par exemple 7,3% d'acide linoléique (oméga-6), de 2 à 5% d'acide palmitique et de 2 à 4% d'acide stéarique mais se distingue surtout par sa teneur en phytostérols de 0,2% à 0,5%, par exemple 0,35% dont une majorité de béta-sitostérol 0,2% et en polyphénols de 0,01 à 0,1%, par exemple 0,074%.

Dans la présente, le profil des stérols peut être mesuré selon tout procédé connu, par exemple selon la norme ISO 1228; le dosage de la Vitamine E peut être mesuré selon tout procédé connu, par exemple selon la norme NF V18-402 ; le profil des acides gras peut être mesuré selon tout procédé connu, par exemple selon la norme NF EN ISO 5508/5509 ; le profil des sucres peut être mesuré par tout procédé connu, par exemple par une technique chromatographique telle que la chromatographie ionique, le dosage des polyphénols totaux peut être mesuré par tout procédé connu, par exemple selon la norme ISO 14502-1.

Dans la présente par « huile essentielle » on entend toute huile essentielle obtenue par tout procédé connu de l'homme du métier. Par exemple, elle peut être obtenue par hydrodistillation. Il peut s'agir par exemple d'une huile essentielle biologique d'immortelle, c'est-à-dire issue de l'agriculture biologique, par exemple une huile essentielle biologique de Corse, par exemple de la société Essences Naturelles Corses.

Dans la présente, l'huile essentielle peut être obtenue, par exemple à partir d'une plante et/ou d'une partie d'une plante. Il peut s'agir par exemple d'une huile obtenue à partir de fleurs ou sommités fleuries, feuilles, graines, racines, tiges.

Dans la présente, l'huile essentielle biologique d'immortelle peut comprendre de 35 à 50 % en poids d'acétate de néryle.

Quelle que soit la nature de l'extrait d'immortelle, les extraits peuvent être obtenus par l'une quelconque des techniques connues de l'homme du métier, par exemple par voie supercritique ou par extraction hydroalcoolique.

Dans la présente par composition cosmétique on entend toute composition à visée cosmétique, par exemple une composition pouvant être mise en contact avec les parties superficielles du corps humain, par exemple l'épiderme, les systèmes pileux et capillaires, les organes externes, les dents et les muqueuses.

Selon l'invention, l'extrait aqueux d'immortelle peut être présent dans la composition par exemple à une concentration de 0,01 à 10% en poids par rapport au poids total de ladite composition, par exemple de 0,01 à 5% en poids par rapport au poids total de ladite composition.

Selon l'invention, l'extrait huileux d'immortelle peut être présent dans la composition par exemple à une concentration de 0,001 à 5% en poids par rapport au poids total de ladite composition, par exemple de 0,001 à 2% en poids par rapport au poids total de la dite composition.

Selon l'invention, l'huile essentielle peut être présente dans la composition de 0,001% à 1% en poids par rapport au poids total de ladite composition, par exemple de 0,001 à 0,5% en poids par rapport au poids total de ladite composition.

Selon l'invention, la somme de l'extrait aqueux, huileux et de l'huile essentielle dans la composition de l'invention peut être de 0,01 à 16% en poids par rapport au poids total de ladite composition, par exemple de 0,01 à 8% en poids par rapport au poids total de ladite composition.

Avantageusement, la composition de l'invention comprenant l'extrait aqueux, huileux et l'huile essentielle d'immortelle permet de renforcer la fonction barrière de la peau. En outre, la composition de l'invention permet avantageusement de favoriser la cohésion entre le derme et l'épiderme, de régénérer et restructurer l'épiderme pour un meilleur lissage en surface et un teint éclatant.

Selon l'invention, quel que soit l'extrait ou le mélanges d'extraits utilisés, la composition cosmétique ou dermatologique de la présente invention peut comprendre en outre au moins un agent actif additionnel choisi, par exemple parmi un agent anti-âge, un agent hydratant, un agent apaisant ou un mélange de ces agents. Ces agents peuvent être ceux couramment utilisés dans les produits cosmétiques et/ou dermatologiques. Des exemples d'agents anti-âges utilisables sont par exemple le silicium ou la vitamine C. Des exemples d'agents hydratants utilisables sont par exemple la glycérine ou l'acide hyaluronique. Des exemples d'agents apaisants utilisables sont par exemple le bisabolol ou l'allantoïne. Ce ou ces agent(s) peuvent être utilisé(s) dans la composition de la présente invention par exemple aux concentrations usuellement utilisées et connues de l'homme du métier dans les compositions cosmétiques ou dermatologiques.

La composition cosmétique selon l'invention peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des agents épaississants minéraux, des agents épaississants organiques, associatifs ou non, des filtres solaires organiques hydrosolubles et liposolubles, des filtres solaires minéraux, des composés siliconés, des parfums, des conservateurs, des céramides, et pseudo-céramides, des vitamines et les provitamines, des protéines, des agents séquestrants, des agents alcanisants, des agents acidifiants, des agents réducteurs, des agents oxydants, des charges minérales, des colorants ou tout autre adjuvant pouvant être cité dans le dictionnaire INCI (International Nomenclature Cosmetic Ingredients) publié par le CTFA (Cosmetic, Toiletry and Fragrance Association).

Selon l'invention, la composition cosmétique et/ou dermatologique peut, par exemple, se présenter sous toute forme connue de l'homme du métier. Il peut s'agir, par exemple d'une émulsion huile-dans-l'eau, eau-dans-l'huile, eau dans silicone, d'une émulsion multiple, d'une mini-émulsion, d'une microémulsion, d'une nano-émulsion, d'une émulsion solide, d'un gel aqueux ou hydro-alcoolique, d'une crème, d'un lait, d'une lotion, d'une pommade, d'une huile, d'un baume, d'un onguent d'un gel, d'un masque, d'une poudre, d'un support imbibé, par exemple un patch transdermique, d'une huile, d'une lotion aqueuse ou hydroalcoolique et/ou une cire.

Selon l'invention, la composition peut être sous toute forme connue de l'homme du métier dans les domaines de la cosmétique et de la dermatologie, sans aucune restriction galénique particulière.

La composition peut être sous toute forme galénique connue de l'homme du métier, par exemple sous la forme d'un onguent, d'une crème, d'une huile, d'un lait, d'une pommade, d'une poudre, d'un stick, d'un tampon imbibé, d'un patch transdermique, d'une solution, d'un gel, d'un spray, d'une lotion ou d'une suspension.

La composition selon la présente invention peut être, par exemple une composition pour le soin du visage, du corps, par exemple des compositions pour le visage et/ou le corps

La composition de l'invention peut être obtenue par tout procédé approprié connu de l'homme du métier pour la fabrication d'une composition cosmétique et/ou dermatologique. Il peut s'agir, par exemple d'un simple mélange. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), procédé classiquement utilisé par l'homme de l'art pour obtenir des émulsions huile dans eau dont les gouttelettes dispersées sont particulièrement fines, par exemple avec un diamètre de 0,1 à 1 μm.

Selon l'invention, la composition peut être une composition utilisée dans le traitement du vieillissement cutanée, par exemple des rides et ridules, de la perte de fermeté, de la perte d'éclat, de la perte d'élasticité et des peaux sèches. Il peut s'agir par exemple d'une composition anti-âge.

La présente invention a également pour objet un procédé de traitement cosmétique anti-âge comprenant l'application sur la peau d'une composition cosmétique comprenant :
- au moins un extrait aqueux d'immortelle,
- au moins un extrait huileux de sommités fleuries d'immortelle, et
- au moins de l'huile essentielle d'immortelle.

L'extrait aqueux, l'extrait huileux et l'huile essentielle sont tels que définis ci-dessus.

Pour cette application, on peut, par exemple, utiliser les formes galéniques décrites ci-dessus. L'application sur la peau peut donc être réalisée en fonction de la forme galénique utilisée.

Il peut s'agir, par exemple d'une simple application sur la peau ou d'une application accompagnée d'un massage de la peau avec la composition de la présente invention.

L'application est de préférence réalisée avec une quantité suffisante de la composition afin que toute la surface de la peau à traiter soit traitée. Il peut s'agir par exemple d'une application classique, comme une crème sur la peau. Il peut s'agir par exemple aussi d'une application pour former un masque traitant.

L'application peut être, par exemple une application quotidienne, hebdomadaire et bi-mensuelle. Il peut s'agir par exemple d'une application une fois par jour, deux fois par jour ou plus.

Avantageusement, la présente invention permet à la fois de renforcer la fonction barrière de la peau ainsi que la cohésion entre le derme et l'épiderme, de régénérer et restructurer l'épiderme pour un meilleur lissage en surface et un teint éclatant.

En outre, la présente invention permet avantageusement de préserver une peau éclatante et de lutter contre les rides en surface, c'est-à-dire en renforçant la couche cornée et en stimulant la différentiation cellulaire et la prolifération cellulaire, ainsi que les rides profondes, c'est-à-dire en renforçant la cohésion entre derme et épiderme.

De plus, les inventeurs ont clairement démontré que la présente invention utilisant l'extrait aqueux, l'extrait huileux et l'huile essentielle d'immortelle agissent de façon synergique sur la régénération et la restructuration de l'épiderme ainsi que sur l'éclat et le teint de la peau.

D'autres caractéristiques et avantages apparaîtront encore à l'homme du métier à la lecture des exemples ci-dessous, donnés à titre illustratif et non limitatif.

### Brève description des figures

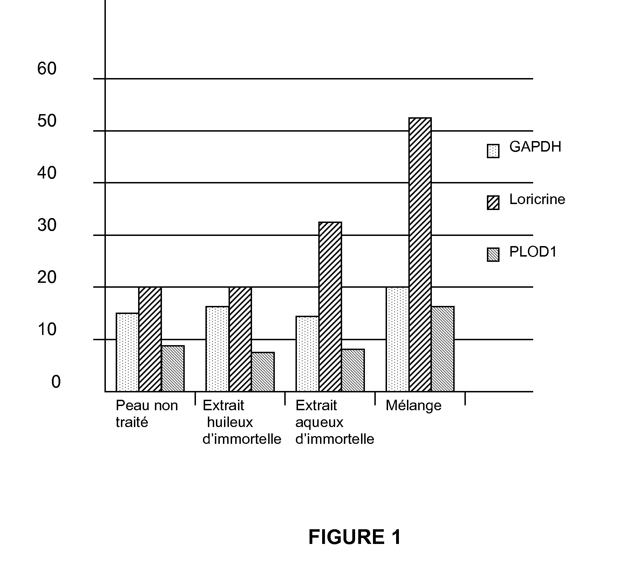

- La figure 1 représente un diagramme en bâton dans lequel l'expression protéique de la Loricrine en pourcentage d'expression par rapport à la GAPDH sur le diagramme et de la procollagen-lysine 1, 2-oxoglutarate 5-dioxygénase 1 (PLOD1) en pourcentage d'expression par rapport à la GAPDH sur le diagramme dans des explants de peau traités avec un extrait huileux d'immortelle (H), un extrait aqueux d'immortelle (A), un mélange de trois extraits d'immortelle (T) par rapport à la peau non traitée (NT) et à l'expression ubiquitaire de la Glycéraldehyde 3-phosphate déhydrogénase (GAPDH).

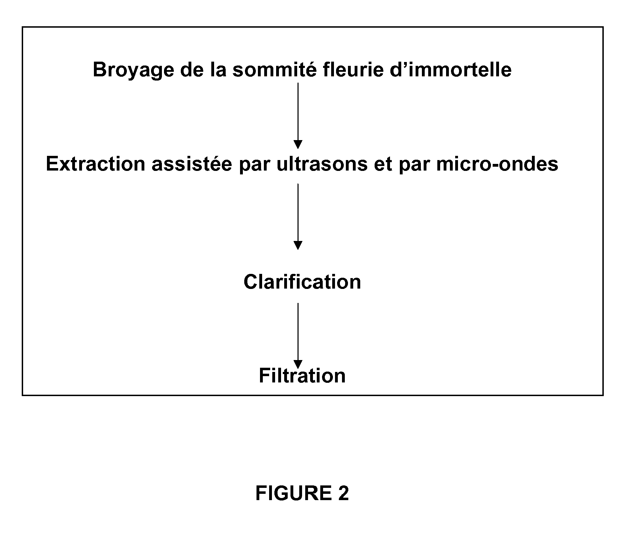

- La figure 2 représente les étapes du procédé d'extraction huileuse.

### EXEMPLES

### Exemple 1 : Exemple de composition selon l'invention pour le soin du visage

Un mélange de base a été réalisé avec différents composants dans une composition de base pour le soin du visage décrite dans le tableau 1 ci-dessous :

**Tableau 1 : Exemple d'une composition de base pour le visage**

| Ingrédients | Quantité en % par rapport au poids total de la composition (%) |
|---|---|
| Stéarate de sucrose | 0,5 à 6% |
| Distéarate de sucrose | 0,5 à 6% |
| Triglycéride caprylique/caprique | 3 à 20% |
| Glycérine | 0,5 à 10% |
| Gomme xanthane | 0,1 à 0,5% |
| Parfum | Qs |
| Conservateurs | Qs |
| Eau | qsp 100% |

A partir de cette composition de base, pour le soin du visage, 18 compositions différentes ont été fabriquées en ajoutant les différents extraits et/ou huiles essentielle d'immortelle dans les proportions indiquées ci-dessous :
Six compositions comprenant un extrait aqueux d'immortelle avec respectivement : 0,01; 0,05; 0,1; 1; 2 et 5% en poids par rapport au poids total de chaque composition ;
Six compositions comprenant l'extrait huileux obtenu par extraction aux micro-ondes d'immortelle avec respectivement: 0,001; 0,01; 0,05; 0,1; 1 et 2% en poids par rapport au poids total de chaque composition ;
Six composition comprenant de l'Huile essentielle d'immortelle avec respectivement : 0,001 ; 0,01 ; 0,02, 0,2 ; 0,5 et 1% en poids par rapport au poids total de chaque composition.

L'extrait aqueux utilisé a été obtenu par extraction aqueuse. Il s'agit d'un extrait aqueux biologique Immortelle de Corse de la société Greentech.

L'extrait huileux utilisé a été obtenu par extraction huileuse par micro-ondes assistée par ultra sons selon procédé décrit dans la figure 2.

L'huile essentielle utilisée a été obtenue par hydrodistillation. Il s'agit d'une huile essentielle biologique d'immortelle de Corse de la société Essences Naturelles Corses.

### Exemple 2: Effet d'un extrait aqueux d'immortelle ou d'un extrait huileux d'immortelle ou de l'huile essentielle d'immortelle et/ou du mélange de l'extrait aqueux, huileux et de l'huile essentielle sur la régénération, restructuration de l'épiderme, le renforcement la fonction barrière, le lissage de la surface de la peau et le renforcement du soutien de la peau

Les expériences ont été réalisées avec un extrait aqueux d'immortelle, un extrait huileux d'immortelle, une huile essentielle d'immortelle et le mélange des trois. L'extrait aqueux bio d'immortelle de Corse a été obtenu par extraction aqueuse et fourni par la société Greentech, l'extrait huileux a été obtenu par extraction micro-ondes en interne et l'huile essentielle d'immortelle a été obtenue par hydrodistillation et fournie par la société Essences Naturelles Corses.

Cet exemple démontre clairement par une analyse transcriptomique *ex-vivo* par Puces à ADN que la composition de l'invention permet de stimuler la régénération et la restructuration de l'épiderme, de renforcer la fonction barrière et l'éclat du teint de la peau.

Le transcriptome est l'ensemble des ARN messager (molécules servant de matrice pour la synthèse des protéines) issu de l'expression d'une partie du génome d'un tissu cellulaire ou d'un type de cellule. La caractérisation et la quantification du transcriptome dans un tissu donné et dans des conditions données permettent d'identifier les gènes actifs, de déterminer les mécanismes de régulation d'expression des gènes et de définir les réseaux d'expression des gènes. Une des techniques utilisées pour mesurer simultanément le niveau d'expression d'un grand nombre de types différents d'ARN messager est celle de la puce à ADN. Les puces à ADN ont permis de mesurer et de visualiser très rapidement les différences d'expression entre les gènes et ceci à l'échelle d'un génome complet.

### 1. Matériel et méthode

### 1.1 Préparation des échantillons :

Des compositions comprenant indépendamment un extrait aqueux, un extrait huileux, une huile essentielle d'immortelle ou le mélange des trois ont été préparées aux différentes concentrations présentées dans l'exemple 1 et utilisées afin d'effectuer des analyses transcriptomiques. Après avoir réalisé un test de viabilité cellulaire tel que décrit dans l'article de Pongsavee.M (Toxicol Ind Health.) [15] qui a permis de déterminer pour chaque composition la concentration maximale non toxique diluée à appliquer,5 explants de peau provenant de lifting du visage d'une patiente âgée de 55 ans ont été incubés indépendamment en présence de ces compositions pendant 24h dans du DMEM (Dulbecco/Vogt modified Eagle's minimal essential medium) sans sérum à 37°C en atmosphère humide à 5% de CO₂. Un explant de peau non traité a été incubé dans les mêmes conditions. Le tableau 1 récapitule les concentrations appliquées pour chaque composition.

**Tableau 2 : concentrations maximales non toxiques exprimées en % pour chaque extrait d'immortelle ou l'huile essentielle ou le mélange de l'extrait aqueux, huileux et de l'huile essentielle d'immortelle**

| **Composition** | **%** |
|---|---|
| extrait aqueux Immortelle | 0,1 |
| extrait huileux Immortelle | 0,02 |
| huile essentielle immortelle | 0,00001 |
| Mélange extrait aqueux, | 0,1 ; |
| extrait huileux, et | 0,02 ; |
| huile essentielle d'immortelle | 0,00001 |

### 1.2 Extraction des ARN totaux et amplification linéaire

Après incubation, les explants de peaux ont été broyés puis les ARN totaux issus de chaque explant ont été extraits en utilisant le RNeasy Kit (Qiagen, Hilden, Germany). Les ARN totaux ont été ensuite quantifiés puis la qualité a été vérifiée en utilisant le BIOanalyzer 2100 avec le RNA 6000 Nano LabChip Kit (Agilent Technologies, Boblingen, Germany). Un RIN entre 7,5 et 10 a attesté de la bonne qualité des ARN totaux issus de chaque explant préalablement incubé ou non avec les différentes compositions précitées

### 1.3 Mesure de l'expression génique sur puce oligonucléotide et acquisition des data :

Les puces oligonucléotides Affymetrix Human Gene 1.0 ST Whole Genome ont été utilisées pour chaque analyse d'expression génique (GeneChip HU Gene 1.0, Affymetrix, Santa Clara, USA).

Une documentation complète ainsi que le protocole expérimental utilisé est décrit dans la publication de Seitz G, Bonin M, Fuchs J et al. Inhibition of glutathione-S-transferase as a treatment strategy for multidrug resistance in childhood rhabdomyosarcoma. International Journal of Oncology 2010 36:491-500*.* [14]) et est disponible sur le site: http://media.affymetrix.com/support/technical/datasheets/gene_1_0_st_datas heet.pdf.

Plus simplement, les cycles de rétrotranscription et d'amplification des ARN totaux en ARNc puis en ADNc ont été réalisés à l'aide des kits Ambion WT Expression (Ambion, Applied Biosystem, USA) et du kit GeneChip WT terminal Labelling and Hybridization User (Affymetrix). L'hybridation des puces et les mesures ont été réalisées par les Microarray Facility Tubingen.

Les Puces ont ensuite été scannées en utilisant le scanner GCS3000 Gene Chip (Affymetrix) et le software GCOS 1.4. Les images scannées ont été ensuite analysées en utilisant la console Expression 1.0 (Affymetrix) afin de convertir des mesures d'intensité lumineuse en données numériques et de normaliser ces données. L'analyse complète des données et une analyse statistique ont été réalisées grâce au logiciel Genespring GX 11 (Agilent Technologies) sur la base, en particulier, d'algorithmes de classification. Des différences d'expression de gènes ainsi obtenues suite à l'application des différentes compositions comprenant indépendamment soit l'extrait aqueux, l'extrait huileux, l'huile essentielle ou le mélange des trois sur des explants de peau par rapport à un explant non traité ont été rassemblées dans le tableau 3 ci-dessous. La valeur de l'expression différentielle de gènes exprimés entre la peau non traitée et la peau traitée avec les différentes compositions précitées présente une p-value < 0,01 (valeur statistique très significative).

**Tableau 3 : différence d'expression génique et signification biologique des gènes suite à l'application des différents extraits d'immortelle et du complexe des 3 sur explants de peau par rapport à un même explant non traité.**

| GENES | dbEST | FONCTIONS | STATUT: stimulation par rapport à la peau non traitée |
|---|---|---|---|
| **LOR Loricrin** | NM_000427.2 | Loricrine, protéine majeure de l'enveloppe cornée - consolidant la barrière cutanée | X4 avec HE Immortelle |
| | | | x2 avec extrait aqueux Immortelle |
| | | | **X8 avec le mélange extrait aqueux, huileux et huile essentielle d'immortelle** |
| **S100A9 S100 calcium binding protein A9** | NM_002965.3 | précurseurs qui régulent la différentiation des kératinocytes en fonction du flux calcique | X 2 avec HE Immortelle |
| | | | **X8 avec le mélange extrait aqueux, huileux et huile essentielle d'immortelle** |
| **S100A7 S100 calcium binding protein A7** | NM_002963.3 | précurseurs qui régulent la différentiation des kératinocytes en fonction du flux calcique | X 5 avec HE Immortelle |
| | | | **X10 avec le mélange extrait aqueux, huileux et huile essentielle d'immortelle** |
| **PLOD1 procollagen-lysine 1, 2-oxoglutarate 5-dioxygenase 1** | NM_000302.2 | stabilise des liaisons intermoléculaires et renforcement de l'architecture globale du derme/épiderme | **X4 avec le mélange extrait aqueux, huileux** et **huile essentielle d'immortelle** |
| **EREG epiregulin** | NM_001432.2 | stimule la prolifération des kératinoyctes donnant un pool de cellules plus important qui vont ensuite se différencier | **X 10 avec le mélange extrait aqueux, huileux et huile essentielle d'immortelle** |
| **ACER1 alkaline ceramidase 1** | NM_133492.2 | enzyme impliqué dans la synthèse des céramides qui avec les shingolipides sont impliqués dans la fonction barrière de la peau | **X8 avec le mélange extrait aqueux, huileux** et **huile essentielle d'immortelle** |
| **LIPM lipase**, **family member M** | NM_001128215.1 | permet la synthèse des Shingolipides permettant au stratum corneum de jouer son rôle de fonction barrière | **X6 avec le mélange extrait aqueux, huileux** et **huile essentielle d'immortelle** |
| **LIPF lipase, family member F** | NM_004190.3 | permet la synthèse des Shingolipides permettant au stratum corneum de jouer son rôle de fonction barrière | **X8 avec le mélange extrait aqueux, huileux et huile essentielle d'immortelle** |
| **LlPK lipase**, **family member K** | NM_001080518.1 | permet la synthèse des Shingolipides permettant au stratum corneum de jouer son rôle de fonction barrière | **X10 avec le mélange extrait aqueux, huileux et huile essentielle d'immortelle** |
| **KLK5 kallikrein-related peptidase 5** | BT006867.1 | ce gène code pour une protéine impliquée dans la desquamation phénomène essentiel pour éviter l'accumulation de cellules mortes en surface | **X3,5 avec le mélange extrait aqueux, huileux et huile essentielle d'immortelle** |
| **SGPL1 sphingosine-1**-**phosphate lyase** 1 | NM_003901.3 | perméabilité de la barrière renforcée et action anti-microbienne/contrôle de la prolifération des kératinocytes | **X2 avec le mélange extrait aqueux, huileux** et **huile essentielle d'immortelle** |

Dans le tableau précité, Xn représente le facteur de stimulation par rapport à la peau non traitée.

Dans le tableau 3 ci-dessus, seule la stimulation a été indiquée, l'absence de stimulation en présence de l'extrait huileux, aqueux ou l'huile essentielle n'a pas été indiquée.

### 1.4 Validation des données d'expression génique par PCR en temps réel

Afin de valider au niveau génique, par une autre méthode, ces données, une expérience de QPCR a été réalisée sur deux gènes cibles LOR et PLOD1 impliqués respectivement dans la fonction barrière et l'architecture globale entre le derme et l'épiderme. Pour cela, des amorces en position 3' et 5 'de chaque gène ont été sélectionnées puis synthétisées afin d'amplifier chaque gène pris séparément. Le tableau 4 regroupe la séquence et les caractéristiques de ces amorces.

**Tableau 4 : séquence et caractéristiques des amorces permettant d'amplifier LOR et PLOD1**

| Amorces | Séquences | SEQ ID NO | TM | %GC |
|---|---|---|---|---|
| LOR L | 5'-CTCACCCTTCCTGGTGCTT-3' | 1 | 60 | 61 |
| LOR R | 5'-GGGTGGGCTGCTTTTTCT-3 | 2 | 60 | 60 |
| PLOD1 L | 5'-TTTTCATCCACAACCACGAG-3 | 3 | 59 | 45 |
| PLOD1 R | 5'-GGTACTCGCTGCCATGCT-3 | 4 | 59 | 61 |
| GAPDH L | 5'-AGCCACATCGCTCAGACAC-3 | 5 | 60 | 58 |
| GAPDH R | 5'-GCCCAATACGACCAAATCC-3 | 6 | 60 | 53 |

Une étape de rétrotranscription a été tout d'abord réalisée en utilisant le système Superscript First-Srand Synthesis System for RT-PCR (Invitrogen Corp, Carlsbad, CA) des ARN issus des explants de peau précédant (Devilard E, Bladou F, Ramuz O, Karsenty G, Dalès JP, Gravis G, Nguyen C, Bertucci F, Xerri L, Birnbaum D. FGFR1 and WT1 are markers of human prostate cancer progression. BMC Cancer. 2006 Nov 30;6:272.[21]).

Les expériences de PCR en temps réel ont été réalisées en utilisant le LightCycler 2.0 Detection (Roche Diagnostic, Suisse) en triplicate avec la GAPDH comme gène de référence et contrôle interne. Le kit d'amplification utilisé dans cette expérience est le FastStart DNA Master plus SYBR Green I Master Mix (Roche Diagnostics, Suisse) (Archambaud C, Sansoni A, Mingueneau M, Devilard E, Delsol G, Malissen B, Malissen M. STAT6 deletion converts the Th2 inflammatory pathology afflicting Lat(Y136F) mice into a lymphoproliferative disorder involving Th1 and CD8 effector T cells. J Immunol. 2009 Mar 1;182(5):2680-9.[22]).

### 1.5 Expression des protéines Loricrine et Plod1 dans les explants de peau traités avec les différents extraits d'immortelle ainsi que le complexe des trois par rapport à un explant non traité.

Afin de démontrer qu'il existe une parfaite corrélation entre ARN et protéine, une expérience de Western Blot (Hohl D, Mehrel T, Lichti U, Turner ML, Roop DR, Steinert PM. Characterization of human loricrin. Structure and function of a new class of epidermal cell envelope proteins. J Biol Chem. 1991 Apr 5;266(10):6626-36.[23]) a été réalisée en utilisant des Anticorps anti Loricrine humaine (sc-133757, Santa Cruz, CA, USA) à la concentration de 1/200 et Plod1 humaine (157H00005351-A01, Abnova, Taipei, TAIWAN) à la concentration de 1/500 avec des anticorps anti Gapdh (SC-47724, Santa Cruz, CA, USA) comme contrôle interne à la concentration de 1/500.

### 2.RESULATS

### 2.1 Profil d'expression génique (« gene profiling »)

### 2.1.1. Impact sur la prolifération cellulaire du complexe composé d'un extrait aqueux d'immortelle, d'un extrait huileux d'immortelle et de l'huile essentielle d'immortelle sur la peau.

Les résultats des analyses d'expression de gènes indiqués dans le tableau 3 ont permis de montrer que l'expression du gène EREG est multipliée par 10 en présence du mélange de l'extrait aqueux, huileux et de l'huile essentielle d'immortelle par rapport à la peau non traitée et par rapport à chaque extrait pris séparément.

Aucune surexpression de ce gène n'a été détectée dans la peau non traitée ainsi que dans la peau traitée respectivement avec indépendamment soit l'extrait aqueux, soit l'extrait huileux ou l'huile essentielle. Ainsi, il existe un réel effet synergique lors du mélange de l'extrait aqueux, l'extrait huileux et l'huile essentielle d'immortelle.

Le gène EREG code pour l'épireguline, un facteur de croissance apparenté à l'EGF (Shirasawa S, Sugiyama S, Baba I, Inokuchi J, Sekine S, Ogino K, Kawamura Y, Dohi T, Fujimoto M, Sasazuki T. Dermatitis due to epiregulin deficiency and a critical role of epiregulin in immune-related responses of keratinocyte and macrophage. Proc Natl Acad Sci USA. 2004 Sep 21;101(38):13921-6 [16] entrainant la prolifération des kératinocytes, signal transmis via ses récepteurs spécifiques : le récepteur épidermique de facteur de croissance (EGFR) et l'Erbb4. Cela donne lieu à un pool de cellules kératinocytaires plus important qui vont ensuite se différencier.

De ce fait, la composition comprenant le mélange de l'extrait aqueux, huileux et l'huile essentielle d'immortelle stimule la prolifération kératinocytaire assurant ainsi une meilleure régénération de l'épiderme et un meilleur lissage de surface en favorisant le renouvellement cellulaire.

### 2.1.2. Impact sur la différenciation épidermique du complexe composé d'un extrait aqueux d'immortelle, d'un extrait huileux d'immortelle et de l'huile essentielle d'immortelle sur la peau.

Les résultats des analyses d'expression génique (tableau 3) permettent de montrer que plusieurs gènes impliqués dans les différents stades de la différenciation épidermique sont surexprimés en présence du mélange de l'extrait aqueux, huileux et l'huile essentielle d'immortelle par rapport à l'extrait aqueux ou huileux ou l'huile essentielle d'immortelle pris séparément et par rapport à la peau non traitée.

Ainsi, il existe un réel effet synergique lié au mélange de l'extrait aqueux, huileux et l'huile essentielle d'immortelle. En effet, l'expression des gènes S100A9 et S100A7 a été respectivement multipliée par 8 et par 10 dans le complexe par rapport à l'huile essentielle d'immortelle où l'expression de ce gène a été multipliée par 2 pour S100A9 et 5 pour S100A7. Aucune stimulation de l'expression de ces gènes n'a été retrouvée dans la peau traitée uniquement avec l'extrait aqueux ou avec l'extrait huileux d'immortelle, ces gènes régulant la différenciation kératinocytaire en fonction du taux calcique dans les cellules.

De plus, le gène codant pour la loricrine (Kim BE, Howell MD, Guttman E, Gilleaudeau PM, Cardinale IR, Boguniewicz M, Krueger JG, Leung DY. TNF-α Downregulates Filaggrin and Loricrin through c-Jun N-terminal Kinase: Role for TNF-α Antagonists to Improve Skin Barrier. J Invest Dermatol. 2011 Feb 24 [17]), protéine essentielle à la formation de l'enveloppe cornée et dont sa composition en acides aminés confère une structure particulièrement insoluble essentielle à la fonction barrière de l'épiderme, voit son expression génique multipliée par 8 avec le mélange par rapport à la peau non traitée. L'expression génique du gène LOR a été multipliée par 2 avec l'extrait aqueux d'immortelle et par 4 avec l'Huile essentielle par rapport à la peau non traitée. De ce fait, il existe un réel effet synergique du mélange comprenant l'extrait aqueux, huileux et l'huile essentielle qui permet de consolider le stratum cornéum, de renforcer la fonction barrière.

Ainsi, tel que démontré ici, le mélange comprenant un extrait aqueux d'immortelle, un extrait huileux d'immortelle et de l'huile essentielle d'immortelle stimule la différenciation kératinocytaire permettant ainsi de restructurer l'épiderme tout en assurant un meilleur lissage de surface.

### 2.1.3. Impact sur la fonction barrière cutanée du complexe composé d'un extrait aqueux d'immortelle, d'un extrait huileux d'immortelle et de l'huile essentielle d'immortelle sur la peau.

Les résultats des analyses d'expression de gènes regroupés dans le tableau 3 ont permis de montrer que l'expression des gènes LIPM, LIPF, LIPK et ACER1 impliqués dans la synthèse des lipides épidermiques constituant un véritable ciment intercellulaire a été multipliée par 10 pour LIPK et par 6 pour LIPM, LIPF et ACER1 en présence du mélange comprenant l'extrait aqueux, huileux et de l'huile essentielle d'immortelle par rapport à la peau non traitée et par rapport à l'extrait aqueux, huileux ou de l'huile essentielle pris séparément.

Aucune surexpression de ces gènes n'a été détectée dans la peau non traitée ainsi que dans la peau traitée respectivement avec soit l'extrait aqueux, soit l'extrait huileux ou l'huile essentielle.

Ainsi, il existe un réel effet synergique du mélange comprenant l'extrait aqueux, huileux et de l'huile essentielle d'immortelle. Ces gènes codent pour des enzymes impliqués dans la synthèse des sphingolipides et céramides (Jungersted JM, Høgh JK, Hellgren LI, Jemec GB, Agner T. Skin barrier response to occlusion of healthy and irritated skin: differences in trans-epidermal water loss, erythema and stratum corneum lipids. Contact Dermatitis. 2010 Dec;63(6):313-9 [18]), permettant de renforcer la fonction barrière de la peau en maintenant l'intégrité de ce ciment intercellulaire.

Tel que démontré ici, le mélange comprenant l'extrait aqueux, huileux et de l'huile essentielle stimule la synthèse des lipides épidermiques permettant ainsi de renforcer la fonction barrière de la peau.

### 2.1.4. Impact sur la desquamation du complexe composé d'un extrait aqueux d'immortelle, d'un extrait huileux d'immortelle et de l'huile essentielle d'immortelle sur la peau.

Les résultats des analyses d'expression de gènes regroupés dans le tableau 3 ont permis de montrer que l'expression du gène KLK5 est multipliée par 3,5 en présence du mélange comprenant l'extrait aqueux, huileux et de l'huile essentielle d'immortelle par rapport à la peau non traitée et par rapport à chaque extrait pris séparément.

Aucune surexpression de ces gènes n'a été détectée dans la peau non traitée ainsi que dans la peau traitée respectivement avec soit l'extrait aqueux, soit l'extrait huileux ou l'huile essentielle. De ce fait, il existe un réel effet synergique du mélange comprenant l'extrait aqueux, huileux et de l'huile essentielle d'immortelle. Le gène KLK5 code pour une enzyme chymotrypsique appartenant à la famille des kalikréines impliquée dans le processus de desquamation de la peau (Meyer-Hoffert U, Wu Z, Schröder JM. Identification of lympho-epithelial Kazal-type inhibitor 2 in human skin as a kallikrein-related peptidase 5-specific protease inhibitor. PLoS One. 2009;4(2):e4372 [19]).

Tel que démontré ici, le mélange comprenant l'extrait aqueux, huileux et de l'huile essentielle stimule le processus de desquamation et permet ainsi de mieux lisser la peau en surface tout en assurant un teint radieux.

### 2.1.5. Impact sur la cohésion cutanée du complexe composé d'un extrait aqueux d'immortelle, d'un extrait huileux d'immortelle et de l'huile essentielle d'immortelle sur la peau.

Les résultats des analyses d'expression de gènes regroupés dans le tableau 3 ont permis de montrer que l'expression du gène PLOD1 est multipliée par 4 en présence du mélange comprenant l'extrait aqueux, huileux et de l'huile essentielle d'immortelle par rapport à la peau non traitée et par rapport à chaque extrait pris séparément.

Aucune surexpression de ces gènes n'a été détectée dans la peau non traitée ainsi que dans la peau traitée respectivement avec soit l'extrait aqueux, soit l'extrait huileux ou l'huile essentielle. De ce fait, il existe un réel effet synergique du mélange comprenant l'extrait aqueux, huileux et de l'huile essentielle d'immortelle. Le gène PLOD1 code pour une enzyme qui stabilise les liaisons intermoléculaires entre fibres de collagène (Takaluoma K, Hyry M, Lantto J, Sormunen R, Bank RA, Kivirikko KI, Myllyharju J, Soininen R. Tissue-specific changes in the hydroxylysine content and cross-links of collagens and altérations in fibril morphology in lysyl hydroxylase 1 knock-out mice. J Biol Chem. 2007 Mar 2;282(9):6588-96 [20]) et fibres d'ancrage constituantes de la matrice extracellulaire et de la jonction dermoépidermique.

Tel que démontré ici, le mélange comprenant l'extrait aqueux, huileux et de l'huile essentielle stimule le bon assemblage des fibres d'ancrage permettant ainsi d'améliorer l'architecture globale de la peau et sa fermeté.

### 2.2 PCR en temps réel

Les résultats de PCR en temps réel permettant de valider sur les gènes LOR et PLOD1 les données de micro-array sont regroupées dans le tableau 5.

**Tableau 5 : expression génique de LOR et PLOD1 par RT-QPCR suite à l'application de l'extrait aqueux, huileux ou de l'huile essentielle ou du mélange des 3 sur explants de peau par rapport à un même explant non traité.**

| GENES/Statut | Extrait aqueux Immortelle | Extrait huileux Immortelle | Huile essentielle Immortelle | Mélange des 3 |
|---|---|---|---|---|
| LOR | x2 | NS | x4 | X10 |
| PLOD1 | NS | NS | NS | X5 |

Dans le tableau 5 ci-dessus « ns » signifie : non significatif

L'expression du gène LOR est multipliée par 2 (x2) dans l'extrait aqueux, par 4 dans l'huile essentielle d'immortelle, multipliée par 10 (x10) dans le mélange des trois. Pour PLOD1, l'expression du gène est multipliée par 5 (x5) dans le mélange des trois, aucune surexpression ne fut retrouvée dans l'extrait aqueux, huileux ou de l'huile essentielle séparément ainsi que dans la peau non traitée.

Les résultats obtenus lors de cette expérience sont identiques à ceux obtenus lors de l'expérience sur puces à ADN ce qui confirme et valide les données de micro-arrays.

### 2.3 Expression protéique

Les résultats des expériences de Western Blot sont reproduits sur la figure 1.

La figure représente expression en protéines de la Loricrine et PLOD1 dans les explants de peau traités avec l'extrait huileux d'immortelle, l'extrait aqueux d'immortelle, le mélange comprenant l'extrait aqueux, huileux et de l'huile essentielle d'immortelle par rapport à la peau non traitée et à l'expression ubiquitaire de la GAPDH

Une surexpression de la Loricrine ainsi que PLOD1 a été retrouvée dans la peau traitée par rapport à la peau non traitée et par rapport à chaque extrait pris séparément. Il existe donc une corrélation entre l'expression génique et protéique.

Tel que démontré ici, le mélange comprenant l'extrait aqueux, huileux et de l'huile essentielle entraine donc une stimulation de l'expression génique de LOR et PLOD1 se traduisant également par une surexpression de leur protéine et de leur fonction. Ceci permet d'améliorer l'architecture globale de la peau, sa fermeté et de restructurer l'épiderme.

### Exemple 3 : Test d'usage et d'efficacité du soin visage crème Précieuse Immortelle anti-âge et évaluation des qualités cosmétiques de cette composition

Pour répondre parfaitement et de façon spécifique au vieillissement avec perte d'éclat, de fermeté et apparition de rides et ridules tel que démontré précédemment les inventeurs ont mis au point un complexe d'actifs synergiques assurant à la fois un effet lissant, raffermissant, restructurant et antiride. De ce fait, la composition de l'invention intitulée « Crème Précieuse » a été appliquée de manière quotidienne pendant 28 jours par 26 femmes entre 36 et 60 ans dans les conditions normales d'utilisation afin d'évaluer ses qualités cosmétiques.

La composition utilisée comprenait les composés suivants :

| Ingrédients | Quantité en % par rapport au poids total de la composition |
|---|---|
| Stéarate de sucrose | 2,5 |
| Distéarate de sucrose | 2,5 |
| Caprylic/capric tryglycéride | 20 |
| Glycérine | 5 |
| Gomme xanthane | 0,1 |
| Parfum | Q.S. |
| Conservateurs | Q.S. |
| Eau | Q.S.P. 100% |
| Huile essentielle d'immortelle | 0,02% |
| Extrait aqueux d'immortelle | 1 % |
| Extrait huileux d'immortelle | 0,5% |

Tous ces volontaires présentaient des rides / ridules ainsi qu'un manque d'éclat et d'élasticité au niveau du visage.
Sur le plan des performances, l'ensemble des résultats obtenus est indiqué dans le tableau 6 ci-dessous :

**Tableau 6 : propriétés cosmétiques de la crème de soin visage Crème Précieuse Immortelle**

| | |
|---|---|
| Effet lissant | induit un effet lissant significatif caractérisé par une diminution de la rugosité moyenne (Ra) de moins de 10% en moyenne. Un effet lissant a été mesuré chez 69% des volontaires |
| Effet antirides | induit un effet antiride significatif caractérisé par une diminution du relief moyen (Rz) : moins 8% en moyenne. Un effet antiride a été mesuré chez 65 % des volontaires. |
| | Une diminution significative de l'amplitude maximale du relief (Rt) de 9 % en moyenne. Un effet antiride a été mesuré chez 73 % des volontaires. |
| Scorage visuel | A induit une amélioration visuelle significative de l'aspect de la peau. Le scorage réalisé par le clinicien a montré : |
| | - une diminution significative du nombre de ridules et de rides : respectivement moins 6 % et moins 7 % en moyenne. Une amélioration de ces paramètres a été observée chez 78 % des volontaires pour les ridules et 77 % des volontaires pour les rides. |
| | - une amélioration significative de l'effet lissant de 28 % en moyenne. Une amélioration de ce paramètre a été observée chez 96 % des volontaires. |

L'évaluation de l'effet lissant a été faite en mesurant le relief cutané à l'aide du PRIMOS COMPACT (marque déposée).

L'évaluation de l'effet antirides a été faite en mesurant le relief cutané à l'aide du PRIMOS COMPACT (marque déposée).

L'évaluation de l'aspect visuel a été faite par scorage dermatologique.

L'étude a été réalisée après 28 jours d'utilisation quotidienne du produit par 27 volontaires.

**Tableau 7 : appréciation des propriétés cosmétiques de la crème de soin visage Crème Précieuse Immortelle par volontaires**

| Evaluation de l'efficacité par appréciation des volontaires (% de satisfaction, réponses "oui tout à fait" / "oui" / "oui un peu") | |
|---|---|
| RESULTATS IMMEDIATS | |
| La peau est souple | 99% |
| La peau est douce | 100% |
| La peau est confortable | 100% |
| La peau est plus éclatante | 91% |
| Le teint est plus frais | 93% |
| Le teint est plus reposé | 93% |
| La texture de la peau paraît visiblement améliorée | 87% |
| La peau est plus belle | 85% |
| La peau est plus ferme | 81% |
| Les rides et ridules sont immédiatement lissées | 76% |
| Les rides sont réduites | 76% |
| La peau est plus lisse | 87% |
| La peau semble retendue | 81% |
| La peau parait plus jeune | 78% |
| La peau semble repulpée | 77% |
| La peau semble plus rebondie | 75% |
| Le visage resplendit de jeunesse | 72% |

| APRES 4 SEMAINES | |
|---|---|
| La peau est souple | 96% |
| La peau est douce | 98% |
| La peau est nourrie en profondeur | 92% |
| La peau semble intensément hydratée | 89% |
| La peau est confortable tout au long de la journée | 96% |
| La peau est plus éclatante | 89% |
| Le teint est plus frais | 91% |
| Le teint est plus reposé | 91% |
| La texture de la peau paraît visiblement améliorée | 87% |
| Le grain de peau est amélioré | 83% |
| La peau est plus belle | 83% |
| La peau est plus ferme | 81% |
| La peau semble restructurée | 79% |
| La peau retrouve son maintien | 77% |
| La structure de l'épiderme est renforcée | 77% |
| Le relâchement cutané est réduit | 74% |
| La peau est fortifiée | 81% |
| La peau est revitalisée | 83% |
| La peau est plus tonique | 79% |
| La peau est reconstruite en profondeur | 74% |
| Les rides et ridules sont immédiatement | 71% |
| lissées | |
| Les rides sont réduites | 63% |
| Les rides sont réparées | 63% |
| Les rides d'expression sont atténuées | 64% |
| Les rides profondes sont corrigées | 60% |
| Les rides profondes sont moins marquées | 70% |
| La peau est plus lisse | 89% |
| La peau semble retendue | 79% |
| La peau parait plus jeune | 73% |
| La longueur des rides diminue | 68% |
| La profondeur des rides diminue | 61% |
| Les rides sont comblées de l'intérieur | 63% |
| La peau semble repulpée | 69% |
| La peau semble plus rebondie | 68% |
| Le visage resplendit de jeunesse | 70% |

Tel que présentés dans le tableau 7, des effets lissants et antirides ont été démontrés par ces études. La composition de l'invention, Crème Précieuse Immortelle a donc été reconnue comme étant très efficace par cette étude clinique ainsi que par les utilisatrices.

### Exemple 4: Test d'Usage et d'efficacité de la Crème Nuit Précieuse Immortelle et évaluation des qualités cosmétiques de cette composition

Afin de valider l'efficacité et l'action de la composition de l'invention, 49 femmes âgées entre 35 et 60 ans (moyenne d'âge est de 49,7 ans) ont été recrutées pour cette étude afin de tester l'activité antiride de cette crème nuit ainsi que les qualités cosmétiques de cette composition.

La composition de base utilisée comprenait les composés suivants :

| Ingrédients | Quantité en % par rapport au poids total de la composition |
|---|---|
| Glyceryl stearate & PEG-100 stearate | 5 |
| Alcool cétylique | 1 |
| Caprylic/capric tryglycéride | 16 |
| Glycerine | 5 |
| Gomme xanthane | 0,1 |
| Parfum | Q.S. |
| Conservateurs | Q.S. |
| Eau | Q.S.P. 100% |
| Huile essentielle d'immortelle | 0,02% |
| Extrait aqueux d'immortelle | 1 % |
| Extrait huileux d'immortelle | 0,5% |

Cette composition est désignée ci-dessous « crème nuit précieuse ».

De ce fait, la composition de la Crème Nuit Précieuse a été appliquée de manière quotidienne pendant 28 jours par 49 femmes dans les conditions normales d'utilisation. Tous ces volontaires présentaient des rides / ridules. Sur le plan des performances antirides, l'ensemble des résultats figure dans le tableau 8 ci-dessous :

**Tableau 8 : propriétés antirides de la Crème Nuit Précieuse Immortelle**

| | | Moyennes et écarts-type (Sd) | | Probabilité p : effet temps test de Wilcoxon | Pourcentage de variation # | Delta |
|---|---|---|---|---|---|---|
| | N | J0 Valeur initiale | J28 | | | |
| Aspect lisse de la peau (aspect visuel - ensemble du visage) | 25 | 4,56±0,96 | 5,32±0,95 | < 0,001 | +17% | +0,76 |
| Fermeté de la peau (au pincement - au niveau de la joue) | 25 | 4,76±0,97 | 5,68±0,90 | < 0,001 | +19% | +0,92 |
| Apparence/intensité globale des rides (aspect visuel - au niveau du contour des yeux) | 25 | 5,48±1,36 | 5,44±1,36 | 1,000 | - | - |
| Apparence / intensité globale des ridules (aspect visuel - au niveau du contour des yeux) | 25 | 5,39±1,18 | 4,97±1,02 | < 0,001 | -8% | -0,42 |
| Profondeur de la ride principale (aspect visuel - au niveau de la patte d'oie) | 25 | 5,93±1,67 | 5,92±1,66 | 1,000 | - | - |
| Nombre de rides (aspect visuel - au niveau de la patte d'oie) | 25 | 3,52±0,70 | 3,48±0,70 | 0,25 | - | - |

Dans le tableau ci-dessus :
« N » correspond au nombre de sujets
« JO » correspond au premier jour de traitement,
« J28 » correspond au 28^{ème} jour de traitement,
« # » correspond aux variations par rapport aux valeurs initiales,
« Delta » correspond au(x) différence(s) entre les moyennes au(x) temps d'évaluation considéré(s) et les valeurs initiales,
« p » correspond au seuil de validité statistique, une valeur de *p<0.05* est très significative ; une valeur de p est proche de la significativité lorsque 0,05 ≤ p ≤ 0,10; une valeur de p ≥ 0,10 est non significative. Le test statistique utilisé pour cette analyse est un test de Student.

Sur le plan des performances, l'ensemble des résultats figure dans le tableau 9 ci-dessous :

**Tableau 9 : Jugement global de la « crème nuit précieuse Immortelle »**

| A L'APPLICATION | |
|---|---|
| La texture s'applique facilement | 96% |
| La texture pénètre rapidement | 96% |
| Le produit laisse un fini non gras | 92% |
| Le produit laisse un fini non collant | 90% |
| Le produit laisse un fini non brillant | 98% |
| La peau est souple | 98% |
| La peau est douce | 98% |
| La peau est immédiatement délassée | 94% |

| AU REVEIL | |
|---|---|
| Les sensations de tiraillement disparaissent | 96% |
| La peau est nourrie en profondeur | 94% |
| La peau est intensément hydratée | 96% |
| La peau est confortable | 96% |
| La peau est réparée | 96% |
| La peau est régénérée | 92% |
| La peau semble renforcée | 92% |
| La peau est éclatante | 86% |
| Le teint est frais | 92% |
| Le teint est reposé | 94% |
| La peau est plus belle | 94% |
| La texture de la peau parait améliorée | 94% |
| La texture de la peau est plus belle | 90% |
| Le grain de peau est amélioré | 82% |
| La peau est plus ferme | 78% |
| La peau semble restructurée | 80% |
| Les traits sont moins marqués | 88% |
| La peau est défroissée | 82% |
| Les rides et ridules sont immédiatement lissées | 61% |
| Les rides sont réduites | 65% |
| Au réveil, la peau est plus lisse | 82% |
| Au réveil, la peau semble retendue | 71% |
| Au réveil, la peau parait plus jeune | 67% |
| Au réveil, la peau semble repulpée | 73% |
| Au réveil, la peau semble plus rebondie | 69% |

| APRES 4 SEMAINES, NUIT APRES NUIT | |
|---|---|
| La peau est souple | 96% |
| La peau est douce | 98% |
| La peau est délassée | 92% |
| Les sensations de tiraillement disparaissent | 90% |
| La peau est nourrie en profondeur | 88% |
| La peau est plus confortable | 92% |
| La peau est réparée | 84% |
| La peau est régénérée | 82% |
| L'épiderme semble profondément réparé | 69% |
| La peau est reconstruite en profondeur | 65% |
| L'épiderme semble renforcé | 73% |
| La peau est éclatante | 80% |
| Le teint est frais | 78% |
| Le teint est reposé | 84% |
| La peau est plus belle | 84% |
| La texture de la peau s'améliore | 92% |
| La peau est plus ferme | 78% |
| La peau semble restructurée | 73% |
| La peau retrouve son maintien | 73% |
| Le relâchement cutané est réduit | 78% |
| La peau est fortifiée | 80% |
| La peau est revitalisée | 80% |
| La peau est plus tonique | 78% |
| Les traits sont moins marqués | 76% |
| La peau est défroissée | 71% |
| Les rides et ridules sont lissées | 69% |

Un jugement positif en tant que crème nuit précieuse et appréciation favorable des volontaires pour les critères suivants (en % des volontaires interrogés - réponses "oui tout à fait" / "oui" / "oui un peu").

La composition comprenant le mélange de l'extrait aqueux, huileux et l'huile essentielle d'immortelle a donc été reconnue comme étant très efficace par les utilisateurs après 4 semaines d'applications.

### Exemple 5 : Test d'Usage et d'efficacité du Fluide Précieux Immortelle et évaluation des qualités cosmétiques de cette composition

Afin de valider l'efficacité et l'action des concentrés actifs spécifiques, 50 femmes âgées entre 35 et 60 ans (moyenne d'âge est de 51 ans) ont été recrutées pour cette étude afin de tester l'activité antiride de cette crème nuit ainsi que les qualités cosmétiques de cette composition. De ce fait, la composition du Fluide Précieux a été appliquée de manière quotidienne pendant 28 jours par 50 femmes dans les conditions normales d'utilisation. Tous ces volontaires présentaient des rides / ridules.

La composition utilisée comprenait les composés suivants :

| Ingrédients | Quantité en % par rapport au poids total de la composition |
|---|---|
| Glycéryl stéarate & PEG-100 stéarate | 1 |
| Alcool cétylique | 1,5 |
| Caprylic/capric tryglycéride | 15 |
| Glycérine | 5 |
| Gomme xanthane | 0,1 |
| Parfum | Q.S. |
| Conservateurs | Q.S. |
| Eau | Q.S.P. 100% |
| Huile essentielle d'immortelle | 0,02% |
| Extrait aqueux d'immortelle | 1 % |
| Extrait huileux d'immortelle | 0,5% |

Cette composition est désignée ci-dessous « fluide précieux».

Sur le plan des performances antirides, l'ensemble des résultats figure dans le tableau 10 ci-dessous :

**Tableau 10 : propriétés antirides du Fluide Précieux Immortelle**

| | | Moyennes et écarts-type (Sd) | | Probabilité p : effet temps test de Wilcoxon | Pourcentage de variation # | Delta |
|---|---|---|---|---|---|---|
| | N | J0 Valeur initiale | J28 | | | |
| Aspect lisse de la peau (aspect visuel - ensemble du visage) | 25 | 4,04±1,24 | 4,60±1,41 | < 0,001 | +14% | +0,56 |
| Fermeté de la peau (au pincement - au niveau de la joue) | 25 | 4,16±0,85 | 4,24±0,83 | 0,500 | - | - |
| Apparence/intensité globale des rides (aspect visuel - au niveau du contour des yeux) | 25 | 4,32±1,46 | 4,24±1,48 | 0,500 | - | - |
| Apparence / intensité globale des ridules (aspect visuel - au niveau du contour des yeux) | 25 | 4,00±1,19 | 3,40±1,00 | < 0,001 | -15% | -0,6 |
| Profondeur de la ride principale (aspect visuel - au niveau de la patte d'oie) | 25 | 4,65±1,90 | 4,42±1,94 | 0,002 | -5% | -0,23 |
| Nombre de rides (aspect visuel - au niveau de la patte d'oie) | 25 | 2,41±0,86 | 2,41±0,86 | 1,000 | - | - |

Dans le tableau ci-dessus :
« N » correspond au nombre de sujets
« J0 » correspond au premier jour de traitement,
« J28 » correspond au 28^{ème} jour de traitement,
« # » correspond aux variations par rapport aux valeurs initiales,
« Delta » correspond au(x) différence(s) entre les moyennes au(x) temps d'évaluation considéré(s) et les valeurs initiales,
« p » correspond au seuil de validité statistique, une valeur de *p<0.05* est très significative ; une valeur de p est proche de la significativité lorsque 0,05 ≤ p ≤ 0,10; une valeur de p ≥ 0,10 est non significative. Le test statistique utilisé pour cette analyse est un test de Student.

Sur le plan des performances, le Fluide Précieux a été reconnu comme étant très efficace par les utilisateurs selon le tableau 11. Après 4 semaines d'utilisation, les actions revendiquées sur le complexe actif sont également validées par les volontaires.

**Tableau 11 : Jugement global du Fluide Précieux Immortelle**

| A L'APPLICATTION | |
|---|---|
| La texture s'applique facilement | 100% |
| La texture pénètre rapidement | 98% |
| Le produit laisse un fini non gras | 96% |
| Le produit laisse un fini non collant | 98% |
| Le produit laisse un fini non brillant | 94% |

| RESULTATS IMMEDIATS | |
|---|---|
| La peau est souple | 96% |
| La peau est douce | 100% |
| La peau est intensément hydratée | 84% |
| La peau est matifiée | 66% |
| La peau est plus éclatante | 80% |
| Le teint est plus frais | 84% |
| Le teint est plus reposé | 72% |
| La texture de la peau paraît visiblement améliorée | 68% |
| La peau est plus belle | 76% |
| La peau est plus ferme | 64% |
| La peau est plus lisse | 68% |
| La peau est retendue | 64% |
| La peau semble repulpée | 54% |

| APRES 4 SEMAINES | |
|---|---|
| La peau est souple | 98% |
| La peau est douce | 98% |
| La peau est veloutée | 86% |
| La peau est intensément hydratée | 76% |
| La peau est matifiée | 62% |
| La peau est plus éclatante | 78% |
| Le teint est plus frais | 80% |
| Le teint est plus reposé | 74% |
| La texture de la peau paraît visiblement améliorée | 72% |
| Le grain de peau est amélioré | 76% |
| La peau est plus belle | 74% |
| La peau est plus ferme | 64% |
| La peau semble restructurée | 58% |
| La peau retrouve son maintien | 62% |
| Le relâchement cutané est réduit | 62% |
| La peau est fortifiée | 64% |
| La peau est revitalisée | 72% |
| La peau est plus tonique | 64% |
| La peau est plus lisse | 80% |
| La peau semble retendue | 70% |
| La peau parait plus jeune | 66% |
| La peau semble repulpée | 60% |

Le Fluide Précieux a donc été reconnu comme étant très efficace par les utilisateurs. Après 4 semaines d'utilisation, les actions revendiquées sur le complexe actif sont validées par les volontaires comme le montre le tableau 10 ci-dessus.

Un jugement positif en tant que Fluide Précieux antirides pour peau normale et mixte et appréciation favorable des volontaires pour les critères suivants (en % des volontaires interrogés - réponses "oui tout à fait" / "oui" / "oui un peu").

Le Fluide Précieux a donc été reconnu comme étant très efficace par les utilisateurs après 4 semaines d'applications.

La composition comprenant le mélange de l'extrait aqueux, huileux et l'huile essentielle d'immortelle a donc été reconnue comme étant très efficace par les utilisateurs après 4 semaines d'applications.

### Exemple 6: Effet d'un extrait aqueux d'immortelle ou d'un extrait huileux d'immortelle ou de l'huile essentielle d'immortelle et/ou du mélange de l'extrait aqueux, huileux et de l'huile essentielle sur la régénération, restructuration de l'épiderme, le renforcement la fonction barrière, le lissage de la surface de la peau et le renforcement du soutien de la peau

Les expériences ont été réalisées avec un extrait aqueux d'immortelle, un extrait huileux d'immortelle, une huile essentielle d'immortelle et le mélange des trois. L'extrait aqueux bio d'immortelle de Corse a été obtenu par extraction aqueuse et fourni par la société Greentech, l'extrait huileux a été obtenu par extraction micro-ondes en interne et l'huile essentielle d'immortelle a été obtenue par hydrodistillation et fournie par la société Essences Naturelles Corses.

Dans cet exemple la mesure de l'expression génique de gènes particulier a été étudiée selon le procédé décrit dans l'exemple 2 précité. En particulier, la préparation des échantillons, l'extraction des ARN totaux, l'amplification linéaire et la mesure de l'expression génique sur puce oligonucléotide et acquisition des données ont été réalisées selon les procédés décrits dans l'exemple 2 précité.

Des différences d'expression de gènes ainsi obtenues suite à l'application d'une composition comprenant indépendamment un extrait aqueux, un extrait huileux, une huile essentielle d'immortelle ou un mélange des trois sur des explants de peau par rapport à un explant non traité ont été rassemblées dans le tableau 12 ci-dessous. La valeur de l'expression différentielle de gènes exprimés entre la peau non traitée et la peau traitée avec les différentes compositions précitées présente une p-value < 0,01 (valeur statistique très significative).

**Tableau 12 : différence d'expression génique et signification biologique des gènes suite à l'application d'une composition comprenant l'extrait aqueux, l'extrait huileux, et l'huile essentielle d'immortelle sur explants de peau par rapport à un même explant non traité.**

| GENES | dbEST | FONCTIONS | STATUT: stimulation par rapport à la peau non traitée |
|---|---|---|---|
| TGM3 Transglutaminase 3 | NM_003245.3 | Liaison (« crosslink ») entre l'involucrine et la loricrine pour former l'enveloppe cornée assurant son insolubilité | X4 avec le mélange extrait aqueux, huileux et huile essentielle d'immortelle |
| TGM1 Transglutaminase 1 | NM_000359.2 | Liaison (« crosslink ») entre l'involucrine et la loricrine pour former l'enveloppe cornée assurant son insolubilité | X2 avec le mélange extrait aqueux, huileux et huile essentielle d'immortelle |
| ELF3 E74 du type facteur 3 (« ELF3 E74 like-factor 3 ») | AF517841.1 | Facteur de transcription induisant spécifiquement l'expression des gènes de la famille SPRR2 | X5 avec le mélange extrait aqueux, huileux et huile essentielle d'immortelle |
| Petite protéine 2C riche en proline («small proline-rich protein 2 C (SPRR2C) » | BC120937.1 | Impliqué dans la formation cornée | X15 avec le mélange extrait aqueux, huileux et huile essentielle d'immortelle |
| Petite protéine 2D riche en proline («small proline-rich protein 2D (SPRR2D) » | NM_006945.4 | Impliqué dans la formation cornée | X16 avec le mélange extrait aqueux, huileux et huile essentielle d'immortelle |

Dans le tableau 12 précité, Xn représente le facteur de stimulation par rapport à la peau non traitée.
Dans le tableau 12 ci-dessus, seule la stimulation a été indiquée, l'absence de stimulation en présence de l'extrait huileux, aqueux ou l'huile essentielle n'a pas été indiquée.

Les résultats obtenus dans le tableau 12 précité montre clairement l'impact sur la différenciation épidermique du mélange composé d'un extrait aqueux d'immortelle, d'un extrait huileux d'immortelle et de l'huile essentielle d'immortelle sur la peau.

Les résultats des analyses d'expression génique, tableau 12 précité permettent de montrer que plusieurs gènes impliqués dans les stades terminaux de différenciation épidermique sont surexprimés en présence du mélange de l'extrait aqueux, huileux et l'huile essentielle d'immortelle par rapport à l'extrait aqueux ou huileux ou l'huile essentielle d'immortelle pris séparément et par rapport à la peau non traitée. Ainsi, il existe un réel effet synergique lié au mélange de l'extrait aqueux, huileux et l'huile essentielle d'immortelle. En effet, l'expression des gènes *TGM3* et *TGM1* a été respectivement multipliée par 4 et par 2 par le mélange comprenant l'huile essentielle d'Immortelle, l'extrait aqueux d'immortelle et l'extrait huileux d'immortelle. Aucune stimulation de l'expression de ces gènes n'a été retrouvée dans la peau traitée uniquement avec, l'huile essentielle d'Immortelle ou avec l'extrait aqueux ou avec l'extrait huileux d'immortelle (résultats non présentés). Ces deux gènes *TGM3* et *TGM1* codent pour des enzymes catalysant des réactions de pontages protéiques entre l'involucrine et la loricrine dont l'expression est stimulée par 8 par le mélange comprenant le mélange comprenant l'huile essentielle d'Immortelle, l'extrait aqueux d'immortelle et l'extrait huileux d'immortelle essentiellement, formant ainsi un complexe protéique macromoléculaire particulièrement stable et insoluble (Russell, L. J., DiGiovanna, J. J., Rogers, G. R., Steinert, P. M., Hashem, N., Compton, J. G., and Bale, S. J. (1995). Mutations in the gene for transglutaminase 1 in autosomal recessive lamellar ichthyosis. Nat Genet, 9(3):279-83 [24]). Ces enzymes ont donc un rôle important dans la différenciation terminale de l'épiderme permettant de consolider le stratum corneum et de renforcer la fonction barrière. De plus, l'expression des gènes *SPRR2C, SPRR2D* et *ELF3 E74* a été respectivement multipliée par 15, par 16 et par 5 par le mélange comprenant l'huile essentielle d'Immortelle, l'extrait aqueux d'immortelle et l'extrait huileux d'immortelle. Aucune stimulation de l'expression de ces gènes n'a été retrouvée dans la peau traitée uniquement avec, l'huile essentielle d'Immortelle ou avec l'extrait aqueux ou avec l'extrait huileux d'immortelle. Les gènes *SPRR2C* et *SPRR2D* (de Koning HD, van den Bogaard EH, Bergboer JG, Kamsteeg M, van Vlijmen-Willems IM, Hitomi K, Henry J, Simon M, Takashita N, Ishida-Yamamoto A, Schalkwijk J, Zeeuwen PL. Expression profile of cornified envelope structural proteins and keratinocyte differentiation-regulating proteins during skin barrier repair. Br J Dermatol. 2012 Feb 13. [25]) sont des constituants de l'enveloppe cornée et présentent une importante homologie de séquences avec la loricrine et l'involucrine. Le gène *ELF3 E74* code pour un facteur de transcription entrainant la stimulation de l'expression des gènes de la famille SPRR2 (Oettgen P, Kas K, Dube A, Gu X, Grall F, Thamrongsak U, Akbarali Y, Finger E, Boltax J, Endress G, Munger K, Kunsch C, Libermann TA. Characterization of ESE-2, a novel ESE-1-related Ets transcription factor that is restricted to glandular epithelium and differentiated keratinocytes. J Biol Chem. 1999 Oct 8;274(41):29439-52. [26]).

Ainsi, tel que démontré ici, le mélange comprenant un extrait aqueux d'immortelle, un extrait huileux d'immortelle et de l'huile essentielle d'immortelle stimule la différenciation terminale kératinocytaire permettant ainsi de restructurer l'épiderme tout en assurant un meilleur lissage de surface. Ceci renforce et confirme les résultats obtenus dans l'exemple 2 précité.

### Exemple 7 : Test d'usage et d'efficacité du soin visage crème baume yeux Immortelle et évaluation des qualités cosmétiques de cette composition

Afin de valider l'efficacité et l'action des concentrés actifs spécifiques, 51 femmes âgées entre 35 et 60 ans (moyenne d'âge était de 50 ans) ont été recrutées pour cette étude afin de tester l'activité antiride de cette crème baume yeux ainsi que les qualités cosmétiques de cette composition. De ce fait, la composition a été appliquée de manière biquotidienne pendant 28 jours par 51 femmes dans les conditions normales d'utilisation. Tous ces volontaires présentaient des rides / ridules.

La composition utilisée comprenait les composés suivants :

| **Ingrédients** | **Quantité en % par rapport au poids total de la composition** |
|---|---|
| Eau | QSP |
| Glycérine | 2 |
| Gélifiant acrylate émulsionnant | 2 |
| Gomme xanthane | 0,15 |
| Carbomer | 0,2 |
| Caprylic/capric triglycérides | 8 |
| Poudres de toucher (polyméthyl méthacrylate Amidon de maïs (« Corn starch »)) | 5 |
| Huile essentielle d'immortelle | 0,01 |
| Extrait aqueux d'immortelle | 0,1 |
| Extrait huileux d'immortelle | 0,01 |

Cette composition est désignée ci-dessous « baume yeux».
Sur le plan des performances une mesure de l'aspect visuel de la peau a été effectuée.

L'évaluation de la fermeté de la peau a été faite en mesurant le relief cutané par scorage clinique. Le médecin en charge de l'étude a effectué une évaluation visuelle en début et en fin d'étude selon une échelle qui s'étend de peau relâchée à peau ferme. L'évaluation de la rugosité de la peau a été faite par scorage clinique. Le médecin en charge de l'étude a effectué une évaluation visuelle en début et en fin d'étude de la rugosité de la peau selon une échelle de peau rugueuse à peau lisse.

Les résultats obtenus figurent dans le tableau 13 ci-dessous.

**Tableau 13 : propriétés antirides de la baume yeux**

| | Moyennes et écarts-type (Sd) | | Pourcentage de variation # | Probabilité p : test de Student | Delta |
|---|---|---|---|---|---|
| | J0 Valeur initiale | J28 | | | |
| Fermeté de la peau (au pincement - au niveau de la joue) | 3,9±0,4 | 4,5±0,40 | 14% | p<0,0001 | 0,5±0,1 |
| Rugosité de la peau | 3,3±0,4 | 4,5±0,30 | 36% | p<0,0001 | 1,2±0,2 |

Dans le tableau 13 ci-dessus :
« N » correspond au nombre de sujets
« J0 » correspond au premier jour de traitement,
« J28 » correspond au 28^{ème} jour de traitement,
« # » correspond aux variations par rapport aux valeurs initiales,
« Delta » correspond au(x) différence(s) entre les moyennes au(x) temps d'évaluation considéré(s) et les valeurs initiales,
« p » correspond au seuil de validité statistique, une valeur de p<0,0001 est très significative ; une valeur de p est proche de la significativité lorsque 0,05 ≤ p ≤ 0,10; une valeur de p ≥ 0,10 est non significative. Le test statistique utilisé pour cette analyse est un test de Student.

Après 28 jours d'utilisation, le baume yeux présente un effet significatif au niveau de la fermeté et du lissage cutané.

Sur le plan des performances, le baume yeux a également été reconnu comme étant très efficace par les utilisateurs selon le tableau 14. Après 4 semaines d'utilisation, les actions revendiquées sur le complexe actif ont donc été validées par les volontaires.

Sur le plan des performances, l'ensemble des résultats figure dans le tableau 14 ci-dessous :

**Tableau 14 : Jugement global de la « baume yeux »**

| A L'APPLICATION | |
|---|---|
| La texture s'applique facilement | 100% |
| La texture pénètre rapidement | 96% |
| Le produit laisse un fini non gras | 88% |
| Le produit laisse un fini non collant | 96% |
| L'application fournit un effet de fraicheur | 84% |
| Le produit laisse un fini velouté | 86% |

| RESULTAT IMMEDIAT | |
|---|---|
| La peau est souple | 90% |
| La peau est douce | 93% |
| La peau est veloutée | 96% |
| La peau est nourrie en profondeur | 84% |
| Le regard est plus lumineux | 73% |
| Le contour de l'oeil est plus frais | 67% |
| Le produit réduit les marques de fatigue | 70% |
| Les cernes sont atténuées | 63% |
| Les poches semblent réduites | 65% |
| Le contour de l'oeil est décongestionné | 74% |
| Le regard est revigoré | 73% |
| Le contour de l'oeil est plus ferme | 70% |
| Le contour de l'oeil est lissé | 76% |
| Les ridules sont réduites | 70% |

| APRES 4 SEMAINES | |
|---|---|
| La peau est souple | 92% |
| La peau est douce | 92% |
| La peau est veloutée | 80% |
| La peau est nourrie en profondeur | 73% |
| La peau est plus confortable | 78% |
| La peau est apaisée | 72% |
| La peau est plus radieuse | 62% |
| Le contour de l'oeil est plus frais | 65% |
| Le produit réduit les marques de fatigue | 65% |
| Les cernes sont atténuées | 65% |
| Le regard est revigoré | 63% |
| Le contour de l'oeil est plus ferme | 66% |
| La structure de la peau est raffermie | 73% |
| Le contour de l'oeil est lissé | 72% |
| Les ridules sont réduites | 69% |
| Les rides d'expressions sont réduites | 69% |
| Les rides de la patte d'oie sont réduites | 67% |
| La peau semble ferme | 61% |
| La regard semble revitalisé | 61% |

Un jugement positif en tant que baume yeux et une appréciation favorable des volontaires pour les critères suivants (en % des volontaires interrogés - réponses "oui tout à fait" / "oui" / "oui un peu") ont été observés.

La composition comprenant le mélange de l'extrait aqueux, huileux et l'huile essentielle d'immortelle a donc été reconnue comme étant très efficace par les utilisateurs après 4 semaines d'applications.

Cet exemple démontre donc clairement l'effet anti-âge d'une composition comprenant un extrait huileux, aqueux et une huile essentielle d'immortelle.

### Listes des références

1. Koblenzer CS. Psychologic aspects of aging and the skin. Clinics Dermatol 1996,14:171-7.
2. Petersen MJ, Hansen C, Craig S. Ultraviolet A irradiation stimulates collagenase production in cultured human fibroblasts. J Invest Dermatol 1992,99:440-4.
3. Pierard GE. The quandary of climacteric skin ageing. Dermatology 1996;193:273-4.
4. West MD. The cellular and molecular biology of skin aging. Arch dermatol 1994;130:87-95*.*
5. Meydani M. Nutrition interventions in aging and age-associated disease. Ann N Y Acad Sci 2001;928:226-35
6. Stege H, Roza L, Vink A et al. Enzyme plus light therapy to repair immunosuppressive effects on human skin damaged by ultraviolet B-radiation. Proc Natl Acad Sci USA 2000; 97:179-85.
7. Jurkiewicz BA, Buettner GR. Ultraviolet light-induced free radical formation in the skin: An electron paramagnetic resonance study. Photochem Photobiol 1994; 59:1-4.
8. Grether-Beck S, Oliazola-Horn S, Schmitt H et al. Activation of transcriptor factor AP-2 mediates UVA radiation- and singlet oxygen-induced expression of the human intercellular adhesion molecule-1 gene. Proc Natl Acad Sci USA 1996; 93:14586-91.
9. Fisher GJ, Wang ZQ, Datta SC, Varani J, Kang S, Voorhees JJ. Pathophysiology of premature skin aging induced by ultraviolet light. N Engl J Med 1997;337:1419-28.
10.Lanmann C, Bergmann J, Harisson G, Young AR. Matrix metalloproteinase-1 and skin aging in smokers. Lancet 2001;357:935-7.
11.Fenske NA, Lober CW. Structural and functional changes of normal aging skin. J Am Acad Dermatol 1986,15:571-85.
12.Harvell JD, Maibach HI. Percutaneous absorption and inflammation in aged skin: a review. J Am Acad Dermatol 1994,31:1015-21.
13.Grove GL, Kligman AM. Age-associated changes in human epidermal cell renewal. J Gerontol 38:137-42.
14.Seitz G, Bonin M, Fuchs J et al. Inhibition of glutathione-S-transferase as a treatment strategy for multidrug resistance in childhood rhabdomyosarcoma. International Journal of Oncology 2010 36:491-500*.*
15.Pongsavee M. In vitro study of lymphocyte antiproliferation and cytogenetic effect by occupational formaldehyde exposure. Toxicol Ind Health. 2011 Apr 19.
16.Shirasawa S, Sugiyama S, Baba I, Inokuchi J, Sekine S, Ogino K, Kawamura Y, Dohi T, Fujimoto M, Sasazuki T. Dermatitis due to epiregulin deficiency and a critical role of epiregulin in immune-related responses of keratinocyte and macrophage. Proc Natl Acad Sci USA. 2004 Sep 21;101 (38):13921-6.
17. Kim BE, Howell MD, Guttman E, Gilleaudeau PM, Cardinale IR, Boguniewicz M, Krueger JG, Leung DY. TNF-α Downregulates Filaggrin and Loricrin through c-Jun N-terminal Kinase: Role for TNF-α Antagonists to Improve Skin Barrier. J Invest Dermatol. 2011 Feb 24.
18. Jungersted JM, Høgh JK, Hellgren LI, Jemec GB, Agner T. Skin barrier response to occlusion of healthy and irritated skin: differences in trans-epidermal water loss, erythema and stratum corneum lipids. Contact Dermatitis. 2010 Dec;63(6):313-9.
19.Meyer-Hoffert U, Wu Z, Schröder JM. Identification of lympho-epithelial Kazal-type inhibitor 2 in human skin as a kallikrein-related peptidase 5-specific protease inhibitor. PLoS One. 2009;4(2):e4372.
20.Takaluoma K, Hyry M, Lantto J, Sormunen R, Bank RA, Kivirikko KI, Myllyharju J, Soininen R. Tissue-specific changes in the hydroxylysine content and cross-links of collagens and alterations in fibril morphology in lysyl hydroxylase 1 knock-out mice. J Biol Chem. 2007 Mar 2;282(9):6588-96.
21.Devilard E, Bladou F, Ramuz O, Karsenty G, Dalès JP, Gravis G, Nguyen C, Bertucci F, Xerri L, Birnbaum D. FGFR1 and WT1 are markers of human prostate cancer progression. BMC Cancer. 2006 Nov 30;6:272.
22.Archambaud C, Sansoni A, Mingueneau M, Devilard E, Delsol G, Malissen B, Malissen M. STAT6 deletion converts the Th2 inflammatory pathology afflicting Lat(Y136F) mice into a lymphoproliferative disorder involving Th1 and CD8 effector T cells. J Immunol. 2009 Mar 1;182(5):2680-9.
23.Hohl D, Mehrel T, Lichti U, Turner ML, Roop DR, Steinert PM. Characterization of human loricrin. Structure and function of a new class of epidermal cell envelope proteins. J Biol Chem. 1991 Apr 5;266(10):6626-36.
24.Russell, L. J., DiGiovanna, J. J., Rogers, G. R., Steinert, P. M., Hashem, N., Compton, J. G., and Bale, S. J. (1995). Mutations in the gene for transglutaminase 1 in autosomal recessive lamellar ichthyosis. Nat Genet, 9(3):279-83.
25.de Koning HD, van den Bogaard EH, Bergboer JG, Kamsteeg M, van Vlijmen-Willems IM, Hitomi K, Henry J, Simon M, Takashita N, Ishida-Yamamoto A, Schalkwijk J, Zeeuwen PL. Expression profile of cornified envelope structural proteins and keratinocyte differentiation-regulating proteins during skin barrier repair. Br J Dermatol. 2012 Feb 13.
26.Oettgen P, Kas K, Dube A, Gu X, Grall F, Thamrongsak U, Akbarali Y, Finger E, Boltax J, Endress G, Munger K, Kunsch C, Libermann TA. Characterization of ESE-2, a novel ESE-1-related Ets transcription factor that is restricted to glandular epithelium and differentiated keratinocytes. J Biol Chem. 1999 Oct 8;274(41):29439-52.

## Revendications

1. Composition cosmétique ou dermatologique comprenant :
- au moins un extrait aqueux d'immortelle,
- au moins un extrait huileux de sommités fleuries d'immortelle, et
- au moins de l'huile essentielle d'immortelle.

2. Composition selon la revendication 1 dans laquelle la concentration de l'extrait aqueux d'immortelle est de 0,01% à 10% en poids rapport au poids total de la composition.

3. Composition selon la revendication 1 ou 2 dans laquelle la concentration en extrait huileux est de 0,001% à 5% en poids rapport au poids total de la composition.

4. Composition selon l'une quelconque des revendications 1 à 3 dans laquelle la concentration en huile essentielle d'immortelle est de 0,001% à 1% en poids rapport au poids total de la composition.

5. Composition selon l'une quelconque des revendications 1 à 4, ladite composition étant sous l'une des formes choisies parmi une crème, un lait, une lotion, une huile, un baume, un onguent un gel, un masque.

6. Utilisation cosmétique d'une composition selon la revendication 1 dans un traitement anti-âge.

7. Procédé de traitement cosmétique anti-âge comprenant l'application sur la peau d'une composition cosmétique selon la revendication 1.

## Patentansprüche

1. Kosmetische oder dermatologische Zusammensetzung, umfassend:
- mindestens einen wässrigen Extrakt der Immortelle,
- mindestens einen Ölextrakt der Blütenspitzen der Immortelle, und
- mindestens ein ätherisches Öl der Immortelle.

2. Zusammensetzung nach Anspruch 1, worin die Konzentration des wässrigen Extrakts der Immortelle 0,01 Gew% bis 10 Gew% bezogen auf das Gesamtgewicht der Zusammensetzung beträgt.

3. Zusammensetzung nach Anspruch 1 oder 2, worin die Konzentration des öligen Extrakts 0,001 Gew% bis 5 Gew% bezogen auf das Gesamtgewicht der Zusammensetzung beträgt.

4. Zusammensetzung nach einem der Ansprüche 1 bis 3, wobei die Konzentration an ätherischem Öl der Immortelle von 0,001 Gew% bis 1 Gew% bezogen auf das Gesamtgewicht der Zusammensetzung beträgt.

5. Zusammensetzung nach einem der Ansprüche 1 bis 4, wobei die Zusammensetzung aus einer der Formen aus einer Creme, einer Milch, einer Lotion, einem Öl, einem Balsam, einer Salbe, einem Gel, einer Maske ausgewählt wird.

6. Kosmetische Verwendung einer Zusammensetzung nach Anspruch 1 in einer Anti-Aging-Behandlung.

7. Verfahren zur kosmetischen Anti-Aging-Behandlung umfassend das Auftragen einer kosmetischen Zusammensetzung nach Anspruch 1 auf die Haut.

## Claims

1. Cosmetic or dermatological composition comprising:
- at least one everlasting aqueous extract
- at least one oil extract of everlasting flower tops, and
- at least of everlasting essential oil.

2. Composition according to claim 1 wherein the concentration of the everlasting aqueous extract is from 0.01% to 10% by weight relative to the total weight of the composition.

3. Composition according to claim 1 or 2 wherein the concentration of the oil extract is from 0.001% to 5% by weight relative to the total weight of the composition.

4. Composition according to any of claim 1 to 3 wherein the concentration of everlasting essential oil is from 0.001% to 1% by weight relative to the total weight of the composition.

5. Composition according to any of claim 1 to 4, said composition being in one of the forms selected from a cream, a milk, a lotion, an oil, balm, ointment, gel, mask.

6. Cosmetic use of a composition according to claim 1 in an anti-aging treatment.

7. An anti-aging cosmetic treatment comprising applying to the skin a cosmetic composition according to claim 1.
